# EUROPÄISCHE PATENTSCHRIFT

(11) **EP 3 294 710 B1**
(45) Veröffentlichungstag und Bekanntmachung des Hinweises auf die Patenterteilung: **12.01.2022**
(21) Anmeldenummer: 16723318.8
(22) Anmeldetag: 10.05.2016
(51) Int. Cl.: C07C 381/04, C08F 12/30, C08F 12/32, C08J 3/24, C08K 5/00, C08K 5/36, C08F 212/08

(54) **MONOMER ZUR COPOLYMERISATION MIT ALKENEN, DIENEN, VINYL-VERBINDUNGEN UND/ODER VINYLIDEN-VERBINDUNGEN, VERFAHREN ZUR HERSTELLUNG EINES COPOLYMERS UND KAUTSCHUKMISCHUNG SOWIE DEREN VERWENDUNG**
MONOMER FOR COPOLYMERIZATION WITH ALKENES, DIENES, VINYL COMPOUNDS AND/OR VINYLIDENE COMPOUNDS, METHOD FOR THE SYNTHESIS OF A COPOLYMER, RUBBER MIXTURE AND USE THEREOF
MONOMÈRE POUR COPOLYMÉRISATION AVEC DES ALCÈNES, DES DIÈNES, DES COMPOSÉS VINYLIQUES ET/OU DES COMPOSÉS DE VINYLIDÈNE, PROCÉDÉ DE PRÉPARATION D'UN COPOLYMÈRE, ET MÉLANGE DE CAOUTCHOUC ET SON UTILISATION

(30) Priorität: 12.05.2015 DE 102015208813
(43) Veröffentlichungstag der Anmeldung: 21.03.2018
(73) Patentinhaber: Continental Reifen Deutschland GmbH, 30165 Hannover (DE)
(72) Erfinder: HERZOG, Katharina, 31177 Harsum (DE); MÜLLER, Lena, 31714 Lauenhagen (DE); RECKER, Carla, 30167 Hannover (DE); PRUSS, Noa, 60598 Frankfurt (DE); CONRAD, Cathrin Sonja, 34346 Hannoversch Münden (DE); VANA, Phillipp, 37581 Bad Gandersheim (DE)
(74) Vertreter: Continental Corporation
(86) Internationale Anmeldenummer: PCT/EP2016/060383
(87) Internationale Veröffentlichungsnummer: WO 2016/180804

(56) Entgegenhaltungen:
- GB-A- 1 355 106
- US-A- 3 686 323
- US-A1- 2003 013 830
- US-A1- 2010 108 239
- ALAN P. KOZIKOWSKI ET AL: "Chemistry of the main group metals: A stereoselective synthesis of allyl vinyl thioethers for the thio-claisen reaction", JOURNAL OF ORGANOMETALLIC CHEMISTRY, Bd. 164, Nr. 3, 1. Januar 1979 (1979-01-01), Seiten C33-C37, XP055167859, ISSN: 0022-328X, DOI: 10.1016/S0022-328X(00)81705-2
- HIEN T. T. DUONG ET AL: "Acid Degradable and Biocompatible Polymeric Nanoparticles for the Potential Codelivery of Therapeutic Agents", MACROMOLECULES, Bd. 44, Nr. 20, 25. Oktober 2011 (2011-10-25), Seiten 8008-8019, XP055282880, US ISSN: 0024-9297, DOI: 10.1021/ma201085z

## Beschreibung

Die Erfindung betrifft ein Monomer zur Copolymerisation mit Alkenen, Dienen, Vinyl-Verbindungen und/oder Vinyliden-Verbindungen, ein Verfahren zur Herstellung eines Copolymers, ein Copolymer, welches durch das Verfahren hergestellt ist, eine schwefelvernetzbare Kautschukmischung sowie die Verwendung der schwefelvernetzbaren Kautschukmischung zur Herstellung von Fahrzeugreifen, Gurten, Riemen und Schläuchen.

Copolymere wie z.B. Styrol-Butadien-Kautschuk werden in schwefelvernetzbaren Kautschukmischungen verwendet um Eigenschaften wie das Abriebverhalten und/oder der Reißeigenschaften und/oder das Rollwiderstandsverhalten zu beeinflussen. Allerdings unterliegt die Struktur der Netzknotenbildung bei der Schwefelvulkanisation weitestgehend dem Zufall. Hierdurch können die genannten Eigenschaften nicht beliebig gezielt beeinflusst und verbessert werden.

Der Erfindung liegt die Aufgabe zu Grunde, Monomere zur Copolymerisation mit Alkenen, Dienen, Vinyl-Verbindungen und/oder Vinyliden-Verbindungen bereitzustellen, sodass mit den hergestellten Copolymeren in einer vulkanisierten Kautschukmischung eine Verbesserung des Abriebverhaltens und/oder der Reißeigenschaften und/oder des Rollwiderstandsverhaltens erzielt wird.

Gelöst wird diese Aufgabe erfindungsgemäß durch ein Monomer gemäß Formel I):

I) A-S-P,

wobei A eine 4-Vinylbenzyl-Gruppe, und
wobei S ein Schwefelatom ist, und
wobei P eine Schutzgruppe ist, die ausgewählt ist aus der Gruppe bestehend aus S(=O)₂-R¹ mit R¹ = Alkyl, Benzyl oder Phenyl und/oder S-C(=S)-N-R²R³ mit R² und R³ = Alkyl, Benzyl oder Phenyl und/oder N-R⁵R⁶ mit R⁵ = Wasserstoffatom (H), Alkyl, Benzyl oder Phenyl und R⁶ = Alkyl, Benzyl oder Phenyl.

Erfindungsgemäß weist die beanspruchte Verbindung somit a) wenigstens eine Doppelbindung, die an einer Polymerisation teilnehmen kann, und b) eine geschützte Mercaptogruppe auf, die später bei der Vulkanisation der damit hergestellten Polymere entschützt wird, sodass das Schwefelatom dieser Mercaptogruppe an der Vulkanisation teilnehmen kann. Hierdurch kann die Netzknotenstruktur der mit dem Copolymer hergestellten Schwefelvulkanisate im Vergleich zum Stand der Technik besser eingestellt werden, was sich positiv auf das Abriebverhalten und/oder der Reißeigenschaften und/oder das Rollwiderstandsverhalten der Kautschukmischung auswirkt.

Die in Formel I) genannten Reste R¹ bis R⁶ der Schutzgruppe P können wie oben aufgeführt Alkylgruppen sein. Hierbei sind 1 bis 10 Kohlenstoffatome bevorzugt. Zudem ist O = Sauerstoffatom, N = Stickstoffatom.

Die Gruppe A ist eine 4-Vinylbenzyl-Gruppe und somit eine chemische Gruppe enthaltend wenigstens eine aliphatische Doppelbindung, und zwar eine endständige Doppelbindung, d.h. vinylische Doppelbindung. Eine derartige Doppelbindung ist geeignet an einer Polymerisation teilzunehmen, womit die Verbindung gemäß Formel I) ein geeignetes Monomer eines späteren Copolymers darstellt.

Unter "Doppelbindung" ist im Rahmen der vorliegenden Erfindung eine Kohlenstoff-Kohlenstoff-Doppelbindung, also C=C, zu verstehen, sofern nicht ausdrücklich anders bezeichnet.

Gemäß einer besonders bevorzugten Ausführungsform der Erfindung handelt es sich bei dem Monomer um 4-Vinylbenzylbenzolthiosulfonat, wobei A eine 4-Vinylbenzyl-Gruppe und
P eine S(=O)₂-R¹ mit R¹ = Phenyl Gruppe ist.

Diese Verbindung weist somit die Formel II) auf

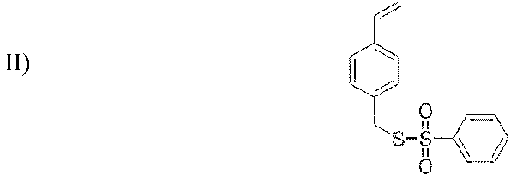

Das Monomer gemäß Formel II), also 4-Vinylbenzylbenzolthiosulfonat (abgekürzt ViBSuT) wird mit folgendem erfindungsgemäßen Verfahren hergestellt:
Die Synthese von ViBSuT erfolgt in stark polaren Lösungsmitteln, wie insbesondere Wasser, Ethanol, DME, da sonst das Natriumbenzolthiosulfonat in einer angemessenen Lösungsmittelmenge nicht vollständig gelöst werden konnte.

Stark polare Lösungsmittel haben bevorzugt eine hohe Dielektrizitätskonstante bzw. dielektrische Leitfähigkeit ε (oder auch Permittivität genannt), infolge dessen sie dissoziierend auf Ionenpaare und Salze wirken. Stark polar Lösungsmittel können protisch sein, d.h. dass sie über eine funktionelle Gruppe verfügen aus der Wasserstoffatome im Molekül als Protonen abgespalten werden können. Beispiele sind Wasser (ε = 78), Methanol, Ethanol und andere Alkohole, Carbonsäuren wie Ameisensäure oder Essigsäure, Ammoniak sowie primäre und sekundäre Amine, primäre und sekundäre Amide wie Formamid (ε = 109), Mineralsäuren wie Schwefelsäure oder Halogenwasserstoffe bzw. Halogenwasserstoffsäuren u.a. Oder aprotische Lösungsmittel, die keine ausreichend aciden Wasserstoffatome, die als Protonen abgespalten werden können. Beispiele sind Ether, Ketone wie Aceton, Nitrile wie Acetonitril, tertiäre Carbonsäureamide wie Dimethylformamid (DMF), Sulfoxide wie Dimethylsulfoxid (DMSO) u.a.

Bevorzugt sind 1,2-Dimethoxyethan (DME), Tetrahydrofuran (THF), Diethylether (DEE), Aceton, Ethanol, Methanol, Wasser, Essigsäure, Dimethylformamid (DMF).

Besonders bevorzugt sind 1,2-Dimethoxyethan (DME), Ethanol, Wasser, Essigsäure, Dimethylformamid (DMF).

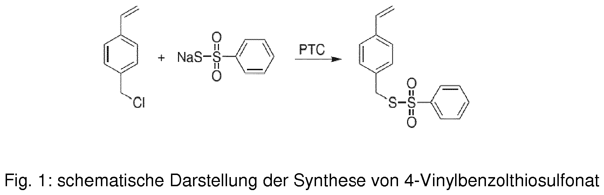

Die Reaktion wird in wässrigen Systemen unter Zuhilfenahme von Phasentransferkatalysatoren (abgekürzt PTC) durchgeführt. Ein Schema ist in Fig. 1 gezeigt.

Toluol als Lösungsmittel für die organische Phase hat den Nachteil niedriger Produktausbeuten.

Die organische Phase besteht somit ausschließlich aus dem Reaktanden 4-Vinylbenzylchlorid. Neben Wasser können Ethanol und 1,2-Dimethoxyethan (DME, Glyme) als stark polare Lösungsmittel in einer phasentransferkatalysierten Reaktion verwendet werden.

### Beispiel 1

Eine Mischung aus frisch destilliertem 4-Vinylbenzylchlorid (9,71 g, 64,0 mmol, 1,0 Äq.), Natriumbenzolthiosulfonat (25,14 g, 128,0 mmol, 2,0 Äq.), Aliquat 336^{®} (1,31 g, 3,2 mmol, 0,05 mol-%) und Wasser (320 mL) wurde bei 65 °C für 380 min gerührt.

Anschließend wurde die Reaktionsmischung auf Raumtemperatur abgekühlt und mit EtOAc (3 x 100 mL) extrahiert. Die vereinigten organischen Phasen wurden mit gesättigter NaCl-Lösung gewaschen, über MgSO₄ getrocknet und im Vakuum eingeengt.

Das Rohprodukt wurde mittels Säulenchromatographie an Kieselgel gereinigt (Hexan/EtOAc 20:1 → 0:1) und das Monomer konnte als weißer Feststoff in 95 %-iger Reinheit isoliert werden (3,11 g, 10,7 mmol, 17 %).

Ein weiterer Gegenstand der vorliegenden Erfindung besteht in einem Verfahren zur Herstellung eines Copolymers durch radikalische Copolymerisation eines oben beschriebenen Monomers mit wenigstens einem weiteren Monomer ausgewählt aus der Gruppe bestehend aus Alkenen und/oder Dienen und/oder Vinyl-Verbindungen und/oder Vinyliden-Verbindungen.

Bei dem erfindungsgemäßen Verfahren zur Herstellung eines Copolymers können alle üblichen radikalischen Verfahren Anwendung finden, insbesondere in Lösung, in Emulsion und unter RAFT-Bedingungen mit RAFT-üblichen Agenzien.

Im Rahmen der vorliegenden Erfindung werden unter Dienen gemäß Römpp Online ungesättigte aliphatische und cycloaliphatische Kohlenwasserstoffe, die im Molekül zwei Doppelbindungen enthalten, verstanden. Bevorzugt sind die beiden Doppelbindungen konjugiert. Bevorzugt ist das konjugierte Dien ausgewählt aus der Gruppe enthaltend, besonders bevorzugt bestehend aus,
1,3-Butadien (Butadien) und/oder 2-Methylbuta-1,3-dien (Isopren = 2-(C₁-C₅-Alkyl)-1,3-Butadien) und/oder 2,3-Dimethyl-1,3-Butadien und/oder 1,3-Pentadien und/oder 2,4-Hexadien und/oder 1,3-Hexadien und/oder 1,3-Heptadien und/oder 1,3-Octadien und/oder 2-Methyl-2,4-Pentadien und/oder Cyclopentadien und/oder 2,4-Hexadien und/oder 1,3-Cyclooctadien und/oder 2-Chlor-1,3-butadien (Chloropren). Besonders bevorzugte Diene sind hierbei Isopren, Butadien und Chloropren.

Alkene, die als Monomere an der Polymerisation beteiligt sein können, sind aliphatische Verbindungen mit einer Doppelbindung, wie insbesondere Ethen, Propen, Buten, Penten, Hexen.

Der Begriff "Vinyl-Verbindung" umfasst im Rahmen der vorliegenden Erfindung alle chemischen Verbindungen, die wenigstens eine Vinyl-Gruppe aufweisen, wie Acrylate, Methacrylate, Acrylsäure, Methacrylsäure, Acrylnitril, sowie vinylaromatische Verbindungen.

Gemäß einer bevorzugten Ausführungsform der Erfindung handelt es sich bei der Vinyl-Verbindung zumindest um wenigstens eine Vinyl-Verbindung, die außer der Vinyl-Gruppe wenigstens eine weitere ungesättigte Gruppe Kohlenstoffgruppe aufweist, wie insbesondere eine Doppelbindung oder einen aromatischen Rest.

Gemäß einer besonders bevorzugten Ausführungsform der Erfindung handelt es sich bei der Vinyl-Verbindung zumindest um wenigstens eine vinylaromatische Verbindung.

Der Begriff "vinylaromatische Verbindung" umfasst im Rahmen der vorliegenden Erfindung monovinylaromatische Verbindungen, d.h. Verbindungen, bei denen nur eine Vinylgruppe an eine aromatische Gruppe gebunden ist, sowie vinylaromatische Verbindungen, bei denen zwei oder mehr Vinylgruppen an eine aromatische Gruppe gebunden sind.

Als vinylaromatische Verbindung ist jede dem Fachmann bekannte denkbar. Bevorzugt ist die vinylaromatische Verbindung ausgewählt aus der Gruppe enthaltend, besonders bevorzugt bestehend aus,
Styrol und/oder C₁₋₄-Alkylsubstituierte Styrole und/oder Stilben und/oder Vinylbenzyldimethylamin und/oder (4-Vinylbenzyl)Dimethylaminoethylether und/oder N,N-Dimethylaminoetyhlstyrol und/oder tert-Butoxystyrol und/oder Vinylpyridin und/oder divinylaromatische Verbindungen.

Bei den C₁₋₄-Alkylsubstituierten Styrolen kann es sich beispielsweise um 2-Methylstyrol und/oder 3-Methylstyrol und/oder 4-Methylstyrol und/oder 2,4-Dimethylstyrol und/oder 2,4,6-Trimethylstyrol und/oder alpha-Methylstyrol und/oder 2,4-Diisopropylstyrol und/oder 4-tert-Butylstyrol handeln.

Der Begriff ^{"}C₁₋₄-Alkylsubstituiert" bedeutet hierbei, dass ein Alkyl-Rest mit 1 bis 4 Kohlenstoffatomen als Substituent eines Wasserstoffatoms vorhanden ist.

Bei den divinylaromatischen Verbindungen kann es sich beispielsweise um 1,2-Divinylbenzol und/oder 1,3-Divinylbenzol und/oder 1,4-Divinylbenzol handeln.

Unter Vinyliden-Verbindungen werden im Rahmen der vorliegenden Erfindung gemäß Römpp Online chemische Verbindungen verstanden, die die über eine Doppelbindung verknüpfte Atomgruppierung R₁R₂=C=CH₂ aufweisen, wie beispielsweise Cl₂C=CH₂ oder F₂C=CH₂.

Gemäß einer Ausführungsform der Erfindung ist es bevorzugt, dass das Alken ausgewählt ist aus der Gruppe bestehend aus Ethen, Propen, Buten, Penten und/oder Hexen, und das Dien ausgewählt ist aus der Gruppe bestehend aus 1,3-Butadien und/oder 2-Methylbuta-1,3-dien und/oder 2,3-Dimethyl-1,3-Butadien und/oder 1,3-Pentadien und/oder 2,4-Hexadien und/oder 1,3-Hexadien und/oder 1,3-Heptadien und/oder 1,3-Octadien und/oder 2-Methyl-2,4-Pentadien und/oder Cyclopentadien und/oder 2,4-Hexadien und/oder 1,3-Cyclooctadien und/oder 2-Chlor-1,3-butadien, und die Vinyl-Verbindung ein Acrylat und/oder Methacrylate und/oder Acrylsäure und/oder Methacrylsäure und/oder Acrylnitril und/oder eine vinylaromatische Verbindung ist, die ausgewählt ist aus der Gruppe bestehend aus Styrol und/oder 2-Methylstyrol und/oder 3-Methylstyrol und/oder 4-Methylstyrol und/oder 2,4-Dimethylstyrol und/oder 2,4,6-Trimethylstyrol und/oder alpha-Methylstyrol und/oder 2,4-Diisopropylstyrol und/oder 4-tert-Butylstyrol und/oder Stilben und/oder Vinylbenzyldimethylamin und/oder (4-Vinylbenzyl)Dimethylaminoethylether und/oder N,N-Dimethylaminoetyhlstyrol und/oder tert-Butoxystyrol und/oder Vinylpyridin und/oder 1,2-Divinylbenzol und/oder 1,3-Divinylbenzol und/oder 1,4-Divinylbenzol.

Das Verfahren zur Herstellung eines Copolymers durch radikalische Copolymerisation ist im Folgenden anhand des Beispiels der Copolymerisation von Verbindung II), also 4-Vinylbenzylbenzolthiosulfonat (abgekürzt ViBSuT), mit Styrol beschrieben.

Das Verfahren ist nicht auf die genannten beispielhaften Monomere beschränkt, sondern kann erfindungsgemäß zur Herstellung aller Copolymere aus den oben beschriebenen Monomeren verwendet werden, inkl. zur Herstellung von Copolymeren aus drei oder mehr verschiedenen Monomeren.

Die Polymerisationsmischung hatte eine Ausgangskonzentration von 3,09 Mol.-% ViBSuT in Bezug auf Styrol (unter Vernachlässigung der Verunreinigungen) sowie 0,05 Mol.-% AIBN. Hierbei ist AIBN die Abkürzung der chemischen Verbindung Azobis(isobutyronitril). Die Polymerisation erfolgte über 6 Stunden bei 60 °C. Das erzielte Copolymer weist ein Zahlenmittel der Molmasse (bzw. der Molmassenverteilung) Mn gemäß GPC von 2350 g/mol und eine Polydispersität (Mw/Mn) von 2,74 auf. Mw stellt hierbei das Gewichtsmittel der Molmassenverteilung gemäß GPC dar.

Mittels Elementaranalyse wird der Schwefelgehalt des hergestellten Copolymers bestimmt, wodurch das Verhältnis der Monomere zueinander bestimmt werden kann, wobei das Vorhandensein großer Restmengen des Monomers 4-Vinylbenzylbenzolthiosulfonat ausgeschlossen werden kann, da insbesondere keine entsprechende Intensität in der Molmassenverteilung zu finden ist. Das gewünschte Copolymer wurde erfolgreich synthetisiert.

Für die Bestimmung der Molmasse bzw. der Molmassenverteilung mittels GPC gelten im Rahmen der vorliegenden Erfindung folgende Messbedingungen: SEC Analysis Systems 1260 Infinity von PSS Agilent mit: PSS Agilent Technologies 1260 Iso Pump G1310B (HPLC-Pumpe), einem Agilent 1260 ALS G1329B Autosampler, einem Agilent 1260 ALS Injektor, einer Vorsäule (PSS SDV, 8 x 50 mm, Partikelgröße 5 µm), drei Trennsäulen (PSS SDV, 8 x 300 mm, Partikelgröße: 5 µm, Porengröße 10⁵ (zehn hoch fünf) Å, 10³ (zehn hoch drei) Ä und 10² (zehn hoch zwei) Ä) und den Detektoren; UV-Detektor PSS Agilent Technologies 1260 VWDVL bei einer Wellenlänge von 310 nm sowie der RI-Detektor PSS Agilent Technologies 1260 RID benutzt; Laufmittel THF (HPLC-grade) mit Toluol (> 99,7 %, trocken) als interner Standard (Fließgeschwindigkeit 1,0 mL/min bei 35 °C). Das System wird mit Polystyrol-Standards mit niedriger Polydispersität von PSS kalibriert. Zur Auswertung wird die Software PSS WinGPC verwendet. Die detektierten Intensitäten werden auf 1 normiert und, sofern nicht anders angegeben, das Signal des RI-Detektors dargestellt.

Ein weiterer Gegenstand der vorliegenden Erfindung besteht in einer schwefelvernetzbaren Kautschukmischung, die wenigstens ein Copolymer enthält, welches durch das Verfahren hergestellt ist. Beispielweise handelt es sich um ein Copolymer aus Styrol und 4-Vinylbenzylbenzolthiosulfonat, welches wie oben beschrieben unter Bedingungen der radikalischen Polymerisation hergestellt wurde.

Bevorzugte Comonomere (neben den Verbindungen I) oder II)) sind oben bereits genannt. Bei dem Copolymer kann es sich auch um ein Polymer aus drei oder mehr verschiedenen Monomeren handeln. Somit kann das Copolymer bspw. ein Terpolymer aus 4-Vinylbenzylbenzolthiosulfonat, Styrol und Butadien sein.

Weiterhin können zwei oder mehr verschiedene Monomere gemäß Formel I) verwendet werden, die z.B. unterschiedliche Schutzgruppen P aufweisen.

Die erfindungsgemäße schwefelvernetzbare Kautschukmischung enthält wenigstens eines der hergestellten Copolymere und kann zudem zusätzlich wenigstens einen im Stand der Technik bekannten Dienkautschuk enthalten.

Als Dienkautschuke werden Kautschuke bezeichnet, die durch Polymerisation oder Copolymerisation von Dienen und/oder Cycloalkenen entstehen und somit entweder in der Hauptkette oder in den Seitengruppen C=C-Doppelbindungen aufweisen.

Bei dem wenigstens einen Dienkautschuk handelt es sich um natürliches Polyisopren und/oder synthetisches Polyisopren und/oder Polybutadien (Butadien-Kautschuk) und/oder unfunktionalisiertes Styrol-Butadien-Copolymer (Styrol-Butadien-Kautschuk) und/oder epoxidiertes Polyisopren und/oder Styrol-Isopren-Kautschuk und/oder HalobutylKautschuk und/oder Polynorbornen und/oder Isopren-Isobutylen-Copolymer und/oder Ethylen-Propylen-Dien-Kautschuk und/oder Nitril-Kautschuk und/oder ChloroprenKautschuk und/oder Acrylat-Kautschuk und/oder Fluor-Kautschuk und/oder SilikonKautschuk und/oder Polysulfid-Kautschuk und/oder Epichlorhydrin-Kautschuk und/oder Styrol-Isopren-Butadien-Terpolymer und/oder hydrierten Acrylnitrilbutadien-Kautschuk und/oder hydrierten Styrol-Butadien-Kautschuk.

Insbesondere Nitrilkautschuk, hydrierter Acrylnitrilbutadienkautschuk, Chloroprenkautschuk, Butylkautschuk, Halobutylkautschuk oder Ethylen-Propylen-Dien-Kautschuk kommen bei der Herstellung von technischen Gummiartikeln, wie Gurte, Riemen und Schläuche, und/oder Schuhsohlen zum Einsatz.

Die Begriffe "vulkanisiert" und "vernetzt" werden im Rahmen der vorliegenden Erfindung synonym verwendet.

Die erfindungsgemäße Kautschukmischung enthält ferner bevorzugt wenigstens einen Füllstoff wie Kieselsäure, Ruß sowie ggf. weitere bekannte polare und/oder unpolare Füllstoffe, wie Alumosilicate, Kreide, Kaolin, Stärke, Magnesiumoxid, Titandioxid und/oder Kautschukgele, sowie Kohlenstoffnanoröhrchen (carbon nanotubes (CNT) inklusive diskreter CNTs, sogenannte hollow carbon fibers (HCF) und modifizierte CNT enthaltend eine oder mehrere funktionelle Gruppen, wie Hydroxy-, Carboxy und CarbonylGruppen) und/oder Graphit und/oder Graphene und/oder sogenannte "carbon-silica dualphase filler".

Handelt es sich bei dem Füllstoff um wenigstens eine Kieselsäure, so enthält die Kautschukmischung bevorzugt 1 bis 300 phr, besonders bevorzugt 1 bis 200 phr, ganz besonders bevorzugt 1 bis150 phr, wenigstens einer Kieselsäure.

Handelt es sich bei dem Füllstoff um wenigstens einen Ruß, so enthält die Kautschukmischung bevorzugt 1 bis 200 phr, besonders bevorzugt 1 bis 170 phr und ganz besonders bevorzugt 1 bis 100 phr wenigstens eines Rußes.

Bei den Kieselsäuren kann es sich um die dem Fachmann bekannten Kieselsäuren, die als Füllstoff für Reifenkautschukmischungen geeignet sind, handeln. Besonders bevorzugt ist es allerdings, wenn eine fein verteilte, gefällte Kieselsäure verwendet wird, die eine Stickstoff-Oberfläche (BET-Oberfläche) (gemäß DIN ISO 9277 und DIN 66132) von 35 bis 350 m²/g, bevorzugt von 60 bis 260 m²/g, besonders bevorzugt von 120 bis 230 m²/g, und eine CTAB-Oberfläche (gemäß ASTM D 3765) von 30 bis 400 m²/g, bevorzugt von 60 bis 250 m²/g, besonders bevorzugt von 120 bis 230 m²/g, aufweist.

Enthält die Kautschukmischung Ruß, sind alle dem Fachmann bekannten Ruß-Typen denkbar. Bevorzugt wird jedoch ein Ruß eingesetzt, der eine Jodadsorptionszahl gemäß ASTM D 1510 von 30 bis 180 g/kg, bevorzugt 30 bis 130 kg/g, und eine DBP-Zahl gemäß ASTM D 2414 von 80 bis 200 ml/100 g, bevorzugt 100 bis 200 ml/100g, besonders bevorzugt 100 bis 180 ml/100g, aufweist.

Die erfindungsgemäße Kautschukmischung kann auch ein Gemisch zweier oder mehrerer der genannten Füllstoffe enthalten.

Zinkoxid gehört im Rahmen der vorliegenden Erfindung nicht zu den Füllstoffen, ist aber bevorzugt in Kombination mit Stearinsäure in der erfindungsgemäßen Kautschukmischung enthalten.

Weiterhin enthält die Kautschukmischung bevorzugt noch weitere Zusatzstoffe.

Weitere Zusatzstoffe beinhaltet im Wesentlichen - neben Zinkoxid (ZnO) und Stearinsäure -ggf. Silan-Kupplungsagenzien für die Anbindung von Kieselsäure an die Polymerketten der enthaltenen Kautschuke, Weichmacher, das Vulkanisationssystem aus Schwefel und/oder Schwefelspendern mit Hilfe von Vulkanisationsbeschleunigern, Ozonschutzmittel, Alterungsschutzmittel, Klebharze, Mastikationshilfsmittel, und neben ZnO und Stearinsäure weitere Aktivatoren bzw. Verarbeitungshilfsmittel, wie z.B. Fettsäuresalze, wie z.B. Zinkseifen und Fettsäureester und deren Derivate, wie z.B. Zinkstearat, oder Zinkkomplexe wie z.B. Zinkethylhexanoat.

Als Silan-Kupplungsagenzien können dabei alle dem Fachmann für die Verwendung in Kautschukmischungen bekannten Silan-Kupplungsagenzien verwendet werden. Hierbei können ein oder mehrere verschiedene Silan-Kupplungsagenzien in Kombination miteinander eingesetzt werden. Die Kautschukmischung kann somit ein Gemisch verschiedener Silane enthalten.

Die Silan-Kupplungsagenzien reagieren mit den oberflächlichen Silanolgruppen der Kieselsäure oder anderen polaren Gruppen während des Mischens des Kautschuks bzw. der Kautschukmischung (in situ) oder bereits vor der Zugabe des Füllstoffes zum Kautschuk im Sinne einer Vorbehandlung (Vormodifizierung). Solche aus dem Stand der Technik bekannten Kupplungsagenzien sind bifunktionelle Organosilane, die am Siliciumatom mindestens eine Alkoxy-, Cycloalkoxy- oder Phenoxygruppe als Abgangsgruppe besitzen und die als andere Funktionalität eine Gruppe aufweisen, die gegebenenfalls nach Spaltung eine chemische Reaktion mit den Doppelbindungen des Polymers eingehen kann. Bei der letztgenannten Gruppe kann es sich z. B. um die folgenden chemischen Gruppen handeln:
-SCN, -SH, -NH₂ oder -Sₓ- (mit x = 2 bis 8).

So können als Silan-Kupplungsagenzien z. B. 3-Mercaptopropyltriethoxysilan, 3-Thiocyanato-propyltrimethoxysilan oder 3,3'-Bis(triethoxysilylpropyl)polysulfide mit 2 bis 8 Schwefelatomen, wie z. B. 3,3'-Bis(triethoxysilylpropyl)tetrasulfid (TESPT), das entsprechende Disulfid (TESPD) oder auch Gemische aus den Sulfiden mit 1 bis 8 Schwefelatomen mit unterschiedlichen Gehalten an den verschiedenen Sulfiden, verwendet werden. TESPT kann dabei beispielsweise auch als Gemisch mit Industrieruß (Handelsname X50S^{®} der Firma Evonik) zugesetzt werden.

Bevorzugt wird ein Silan-Gemisch eingesetzt, welches zu 40 bis 100 Gew.-% Disulfide, besonders bevorzugt 55 bis 85 Gew.-% Disulfide und ganz besonders bevorzugt 60 bis 80 Gew.-% Disulfide enthält. Ein solches Gemisch ist z.B. unter dem Handelsnamen Si 261^{®} der Firma Evonik erhältlich, welches z.B. in der DE 102006004062 A1 beschrieben ist. Auch geblockte Mercaptosilane, wie sie z. B. aus der WO 99/09036 bekannt sind, können als Silan-Kupplungsagens eingesetzt werden. Auch Silane, wie sie in der WO 2008/083241 A1, der WO 2008/083242 A1, der WO 2008/083243 A1 und der WO 2008/083244 A1 beschrieben sind, können eingesetzt werden. Verwendbar sind z. B. Silane, die unter dem Namen NXT (z.B. 3-(Octanoylthio)-1-Propyl-Triethoxysilan) in verschiedenen Varianten von der Firma Momentive, USA, oder solche, die unter dem Namen VP Si 363^{®} von der Firma Evonik Industries vertrieben werden.

Ferner ist es denkbar, dass eines der oben genannten Mercaptosilane, insbesondere 3-Mercaptopropyltriethoxysilan, in Kombination mit Verarbeitungshilfsmitteln (die unten aufgeführt sind), insbesondere PEG-Carbonsäureester, eingesetzt werden.

Weiterhin kann die Kautschukmischung weitere Aktivatoren und/oder Agenzien für die Anbindung von Füllstoffen, insbesondere Ruß, enthalten. Hierbei kann es sich beispielsweise um die z.B. in der EP 2589619 A1 offenbarte Verbindung S-(3-Aminopropyl)Thioschwefelsäure und/oder deren Metallsalze handeln, wodurch sich insbesondere bei der Kombination mit wenigstens einem Ruß als Füllstoff sehr gute physikalische Eigenschaften der Kautschukmischung ergeben.

Der Mengenanteil der Gesamtmenge an weiteren Zusatzstoffen beträgt 3 bis 150 phr, bevorzugt 3 bis 100 phr und besonders bevorzugt 5 bis 80 phr.

Zu den im Rahmen der vorliegenden Erfindung verwendeten Weichmachern gehören alle dem Fachmann bekannten Weichmacher wie aromatische, naphthenische oder paraffinische Mineralölweichmacher, wie z.B. MES (mild extraction solvate) oder RAE (Residual Aromatic Extract) oder TDAE (treated distillate aromatic extract), oder Rubberto-Liquid-Öle (RTL) oder Biomass-to-Liquid-Öle (BTL) bevorzugt mit einem Gehalt an polyzyklischen Aromaten von weniger als 3 Gew.-% gemäß Methode IP 346 oder Rapsöl oder Faktisse oder Weichmacherharze, die nicht zu den oben genannten Klebharzen zählen, oder Flüssig-Polymere, deren mittleres Molekulargewicht (Bestimmung per GPC = gel permeation chromatography, in Anlehnung an BS ISO 11344:2004) zwischen 500 und 20000 g/mol liegt. Werden in der erfindungsgemäßen Kautschukmischung zusätzliche Flüssig-Polymere als Weichmacher eingesetzt, so gehen diese nicht als Kautschuk in die Berechnung der Zusammensetzung der Polymermatrix ein.

Die in dieser Schrift verwendete Angabe phr (parts per hundred parts of rubber by weight) ist dabei die in der Kautschukindustrie übliche Mengenangabe für Mischungsrezepturen. Die Dosierung der Gewichtsteile der einzelnen Substanzen wird dabei stets auf 100 Gewichtsteile der gesamten Masse aller in der Mischung vorhandenen Kautschuke bezogen.

Die Vulkanisation der erfindungsgemäßen schwefelvernetzbaren Kautschukmischung wird in Anwesenheit von Schwefel und/oder Schwefelspendern mit Hilfe von Vulkanisationsbeschleunigern durchgeführt, wobei einige Vulkanisationsbeschleuniger zugleich als Schwefelspender wirken können. Dabei ist der Beschleuniger ausgewählt aus der Gruppe bestehend aus Thiazolbeschleunigern und/oder Mercaptobeschleunigern und/oder Sulfenamidbeschleunigern und/oder Thiocarbamatbeschleunigern und/oder Thiurambeschleunigern und/oder Thiophosphatbeschleunigern und/oder Thiohamstoffbeschleunigern und/oder Xanthogenat-Beschleunigern und/oder Guanidin-Beschleunigern.

Bevorzugt ist die Verwendung eines Sulfenamidbeschleunigers, der ausgewählt ist aus der Gruppe bestehend aus N-Cyclohexyl-2-benzothiazolsufenamid (CBS) und/oder N,N-Dicyclohexylbenzothiazol-2-sulfenamid (DCBS) und/oder Benzothiazyl-2-sulfenmorpholid (MBS) und/oder N-tert-Butyl-2-benzothiazylsulfenamid (TBBS).

Als schwefelspendende Substanz können dabei alle dem Fachmann bekannten schwefelspendenden Substanzen verwendet werden. Enthält die Kautschukmischung eine schwefelspendende Substanz, ist diese bevorzugt ausgewählt aus der Gruppe enthaltend z.B. Thiuramdisulfide, wie z.B. Tetrabenzylthiuramdisulfid (TBzTD) und/oder Tetramethylthiuramdisulfid (TMTD) und/oder Tetraethylthiuramdisulfid (TETD), und/oder Thiuramtetrasulfide, wie z.B. Dipentamethylenthiuramtetrasulfid (DPTT), und/oder Dithiophosphate, wie z.B.

DipDis (Bis-(Diisopropyl)thiophosphoryldisulfid) und/oder Bis(O,O-2-ethylhexylthiophosphoryl)Polysulfid (z. B. Rhenocure SDT 50^{®}, Rheinchemie GmbH) und/oder Zinkdichloryldithiophosphat (z. B. Rhenocure ZDT/S^{®}, Rheinchemie GmbH) und/oder Zinkalkyldithiophosphat, und/oder 1,6-Bis(N,N-dibenzylthiocarbamoyldithio)hexan und/oder Diarylpolysulfide und/oder Dialkylpolysulfide.

Auch weitere netzwerkbildende Systeme, wie sie beispielsweise unter den Handelsnamen Vulkuren^{®}, Duralink^{®} oder Perkalink^{®} erhältlich sind, oder netzwerkbildende Systeme, wie sie in der WO 2010/049216 A2 beschrieben sind, können in der Kautschukmischung eingesetzt werden.

Ein weiterer Gegenstand der der vorliegenden Erfindung besteht in der Verwendung der beschriebenen schwefelvernetzbaren Kautschukmischung zur Herstellung eines Fahrzeugreifens. Damit ist ein weiterer Gegenstand der Erfindung auch ein Fahrzeugreifen, bei dessen Herstellung wenigstens eine erfindungsgemäße schwefelvernetzbare Kautschukmischung - enthaltend wenigstens ein erfindungsgemäßes Copolymer hergestellt mit wenigstens einem erfindungsgemäßen Verfahren wie oben beschrieben - verwendet wurde.

Unter Fahrzeugreifen werden im Rahmen der vorliegenden Erfindung Fahrzeugluftreifen und Vollgummireifen, inklusive Reifen für Industrie- und Baustellenfahrzeuge, LKW-, PKW- sowie Zweiradreifen verstanden.

Gemäß einer bevorzugten Ausführungsform der Erfindung handelt es sich um einen Fahrzeugluftreifen.

Hierbei ist die Verwendung in allen Reifenbauteilen prinzipiell denkbar, wie insbesondere dem Laufstreifen und/oder der Seitenwand und/oder in wenigstens einem inneren Bauteil.

Als innere Reifenbauteile werden im Wesentlichen Squeegee, Innenseele (Innenschicht), Kernprofil, Gürtel, Schulter, Gürtelprofil, Karkasse, Wulstverstärker, Wulstprofil, Hornprofil und Bandage bezeichnet.

Kautschukmischungen der inneren Reifenbauteile sowie der Seitenwand werden auch als Body-Mischung bezeichnet.

Bevorzugt wird die erfindungsgemäße Kautschukmischung jedoch in Laufstreifen von Fahrzeugreifen verwendet, bevorzugt hierbei wenigstens in der Cap von Laufstreifen mit CapB ase- Konstruktion.

Laufstreifen tragen in erheblichem Maße zum Abriebverhalten und dem Rollwiderstand des Fahrzeugreifens bei. Zudem müssen insbesondere Laufstreifen rissbeständig sein.

Zur Verwendung in Fahrzeugreifen wird die Mischung als Fertigmischung vor der Vulkanisation bevorzugt in die Form eines Laufstreifens, bevorzugt wenigstens in die Form einer Laufstreifencap, gebracht und bei der Herstellung des Fahrzeugreifenrohlings wie bekannt aufgebracht. Der Laufstreifen, bevorzugt wenigstens die Laufstreifencap, kann aber auch in Form eines schmalen Kautschukmischungsstreifens auf einen Reifenrohling aufgewickelt werden. Bei zweigeteilten Laufstreifen (oberer Teil: Cap und unterer Teil: Base) kann die erfindungsgemäße Kautschukmischung sowohl für die Cap als auch für die Base verwendet werden.

Die Herstellung der erfindungsgemäßen Kautschukmischung zur Verwendung als Body-Mischung in Fahrzeugreifen erfolgt wie bereits für den Laufstreifen beschrieben. Der Unterschied liegt in der Formgebung nach dem Extrusionsvorgang bzw. dem Kalandrieren der Mischung. Die so erhaltenen Formen der noch unvulkanisierten Kautschukmischung für eine oder mehrere unterschiedliche Body-Mischungen dienen dann dem Aufbau eines Reifenrohlings.

Der Reifenrohling wird anschließend unter im Stand der Technik bekannten Bedingungen vulkanisiert.

Ein weiterer Gegenstand der vorliegenden Erfindung besteht in der Verwendung der beschriebenen schwefelvernetzbaren Kautschukmischung zur Herstellung eines Gurtes, Riemens oder Schlauches.

Zur Verwendung der erfindungsgemäßen Kautschukmischung in Riemen, Gurten und Schläuchen, insbesondere in Fördergurten, wird die extrudierte noch unvulkanisierte Mischung in die entsprechende Form gebracht und ggf. dabei oder nachher häufig mit Festigkeitsträgern, z.B. synthetische Fasern oder Stahlcorde, versehen. Zumeist ergibt sich so ein mehrlagiger Aufbau, bestehend aus einer und/oder mehrerer Lagen Kautschukmischung, einer und/oder mehrerer Lagen gleicher und/oder verschiedener Festigkeitsträger und einer und/oder mehreren weiteren Lagen dergleichen und/oder einer anderen Kautschukmischung.

## Patentansprüche

1. Monomer gemäß Formel I) zur Copolymerisation mit Alkenen, Dienen, Vinyl-Verbindungen und/oder Vinyliden-Verbindungen
I) A-S-P,
wobei A eine 4-Vinylbenzyl-Gruppe ist, und
wobei S ein Schwefelatom ist, und
wobei P eine Schutzgruppe ist, die ausgewählt ist aus der Gruppe bestehend aus S(=O)₂-R¹ mit R¹ = Alkyl, Benzyl oder Phenyl und/oder S-C(=S)-N-R²R³ mit R² und R³ = Alkyl, Benzyl oder Phenyl und/oder N-R⁵R⁶ mit R⁵ = Wasserstoffatom (H), Alkyl, Benzyl oder Phenyl und
R⁶ = Alkyl, Benzyl oder Phenyl.

2. Monomer nach Anspruch 1, **dadurch gekennzeichnet, dass** es sich um 4-Vinylbenzylbenzolthiosulfonat handelt.

3. Verfahren zur Herstellung eines Copolymers durch radikalische Copolymerisation eines Monomers nach einem der Ansprüche 1 oder 2 mit wenigstens einem weiteren Monomer ausgewählt aus der Gruppe bestehend aus Alkenen und/oder Dienen und/oder Vinyl-Verbindungen und/oder Vinyliden-Verbindungen.

4. Verfahren nach Anspruch 3, **dadurch gekennzeichnet, dass** das Alken ausgewählt ist aus der Gruppe bestehend aus Ethen, Propen, Buten, Penten und/oder Hexen, und
das Dien ausgewählt ist aus der Gruppe bestehend aus 1,3-Butadien und/oder 2-Methylbuta-1,3-dien und/oder 2,3-Dimethyl-1,3-Butadien und/oder 1,3-Pentadien und/oder 2,4-Hexadien und/oder 1,3-Hexadien und/oder 1,3-Heptadien und/oder 1,3-Octadien und/oder 2-Methyl-2,4-Pentadien und/oder Cyclopentadien und/oder 2,4-Hexadien und/oder 1,3-Cyclooctadien und/oder 2-Chlor-1,3-butadien, und
die Vinyl-Verbindung ein Acrylat und/oder Methacrylate und/oder Acrylsäure und/oder Methacrylsäure und/oder Acrylnitril und/oder eine vinylaromatische Verbindung ist, die ausgewählt ist aus der Gruppe bestehend aus Styrol und/oder 2-Methylstyrol und/oder 3-Methylstyrol und/oder 4-Methylstyrol und/oder 2,4-Dimethylstyrol und/oder 2,4,6-Trimethylstyrol und/oder alpha-Methylstyrol und/oder 2,4-Diisopropylstyrol und/oder 4-tert-Butylstyrol und/oder Stilben und/oder Vinylbenzyldimethylamin und/oder (4-Vinylbenzyl)Dimethylaminoethylether und/oder N,N-Dimethylaminoetyhlstyrol und/oder tert-Butoxystyrol und/oder Vinylpyridin und/oder 1,2-Divinylbenzol und/oder 1,3-Divinylbenzol und/oder 1,4-Divinylbenzol.

5. Schwefelvernetzbare Kautschukmischung **dadurch gekennzeichnet, dass** sie wenigstens ein Copolymer enthält, welches durch ein Verfahren gemäß einem der Ansprüche 3 oder 4 hergestellt ist.

6. Verwendung einer schwefelvernetzbaren Kautschukmischung nach Anspruch 5 zur Herstellung eines Fahrzeugreifens.

7. Verwendung einer schwefelvernetzbaren Kautschukmischung nach Anspruch 5 zur Herstellung eines Gurtes, Riemens oder Schlauches.

8. Verfahren zur Herstellung von 4-Vinylbenzylbenzolthiosulfonat durch Reaktion von 4-Vinylbenzylchlorid mit Natriumbenzolthiosulfonat in stark polaren Lösungsmitteln, insbesondere Wasser, unter Zuhilfenahme von Phasentransferkatalysatoren.

## Claims

1. Monomer of formula I) for copolymerization with alkenes, dienes, vinyl compounds and/or vinylidene compounds
I) A-S-P
where A is a 4-vinylbenzyl group, and
where S is a sulfur atom, and
where P is a protecting group selected from the group consisting of S(=O)₂-R¹ with R¹ = alkyl, benzyl or phenyl and/or S-C (=S) -N-R²R³ with R² and R³ = alkyl, benzyl or phenyl and/or N-R⁵R⁶ with R⁵ = hydrogen atom (H), alkyl, benzyl or phenyl and
R⁶ = alkyl, benzyl or phenyl.

2. Monomer according to Claim 1, **characterized in that** it is
4-vinylbenzyl benzenethiosulfonate.

3. Process for preparing a copolymer by free-radical copolymerization of a monomer according to either of Claims 1 and 2 with at least one further monomer selected from the group consisting of alkenes and/or dienes and/or vinyl compounds and/or vinylidene compounds.

4. Process according to Claim 3, **characterized in that** the alkene is selected from the group consisting of ethene, propene, butene, pentene and/or hexene, and the diene is selected from the group consisting of 1,3-butadiene and/or
2-methylbuta-1,3-diene and/or 2,3-dimethyl-1,3-butadiene and/or 1,3-pentadiene and/or 2,4-hexadiene and/or 1,3-hexadiene and/or 1,3-heptadiene and/or 1,3-octadiene and/or 2-methyl-2,4-pentadiene and/or cyclopentadiene and/or 2,4-hexadiene and/or 1,3-cyclooctadiene and/or 2-chloro-1,3-butadiene, and
the vinyl compound is an acrylate and/or methacrylates and/or acrylic acid and/or methacrylic acid and/or acrylonitrile and/or a vinylaromatic compound selected from the group consisting of styrene and/or 2-methylstyrene and/or 3-methylstyrene and/or 4-methylstyrene and/or 2,4-dimethylstyrene and/or 2,4,6-trimethylstyrene and/or alpha-methylstyrene and/or 2,4-diisopropylstyrene and/or 4-tert-butylstyrene and/or stilbene and/or vinylbenzyldimethylamine and/or 4-vinylbenzyl dimethylaminoethyl ether and/or N,N-dimethylaminoethylstyrene and/or tert-butoxystyrene and/or vinylpyridine and/or 1,2-divinylbenzene and/or 1,3-divinylbenzene and/or 1,4-divinylbenzene.

5. Sulfur-crosslinkable rubber mixture, **characterized in that** it comprises at least one copolymer which has been prepared by a process according to either of Claims 3 and 4.

6. Use of a sulfur-crosslinkable rubber mixture according to Claim 5 for production of a motor vehicle tire.

7. Use of a sulfur-crosslinkable rubber mixture according to Claim 5 for production of a cord, belt or hose.

8. Process for preparing 4-vinylbenzyl benzenethiosulfonate by reaction of
4-vinylbenzyl chloride with sodium benzenethiosulfonate in strongly polar solvents, especially water, with the aid of phase transfer catalysts.

## Revendications

1. Monomère selon la formule I) pour la copolymérisation avec des alcènes, des diènes, des composés de vinyle et/ou des composés de vinylidène
I) A-S-P,
dans laquelle A est un groupe 4-vinylbenzyle, et
dans laquelle S est un atome de soufre, et
dans laquelle P est un groupe protecteur, qui est choisi dans le groupe constitué par S(=O)₂-R¹ avec R¹ = alkyle, benzyle ou phényle et/ou S-C (=S) -N-R²R³ avec R² et R³ = alkyle, benzyle ou phényle et/ou
N-R⁵R⁶ avec R⁵ = atome d'hydrogène (H), alkyle, benzyle ou phényle et R⁶ = alkyle, benzyle ou phényle.

2. Monomère selon la revendication 1, **caractérisé en ce qu'**il s'agit du benzènethiosulfonate de 4-vinylbenzyle.

3. Procédé de fabrication d'un copolymère par copolymérisation radicalaire d'un monomère selon l'une quelconque des revendications 1 ou 2 avec au moins un autre monomère choisi dans le groupe constitué par les alcènes et/ou les diènes et/ou les composés de vinyle et/ou les composés de vinylidène.

4. Procédé selon la revendication 3, **caractérisé en ce que** l'alcène est choisi dans le groupe constitué par l'éthène, le propène, le butène, le pentène et/ou l'hexène, et
le diène est choisi dans le groupe constitué par le 1,3-butadiène et/ou le 2-méthylbuta-1,3-diène et/ou le 2,3-diméthyl-1,3-butadiène et/ou le 1,3-pentadiène et/ou le 2,4-hexadiène et/ou le 1,3-hexadiène et/ou le 1,3-heptadiène et/ou le 1,3-octadiène et/ou le 2-méthyl-2,4-pentadiène et/ou le cyclopentadiène et/ou le 2,4-hexadiène et/ou le 1,3-cyclooctadiène et/ou le 2-chloro-1,3-butadiène, et
le composé de vinyle est un acrylate et/ou un méthacrylate et/ou l'acide acrylique et/ou l'acide méthacrylique et/ou l'acrylonitrile et/ou un composé aromatique de vinyle, qui est choisi dans le groupe constitué par le styrène et/ou le 2-méthylstyrène et/ou le 3-méthylstyrène et/ou le 4-méthylstyrène et/ou le 2,4-diméthylstyrène et/ou le 2,4,6-triméthylstyrène et/ou l'alpha-méthylstyrène et/ou le 2,4-diisopropylstyrène et/ou le 4-tert-butylstyrène et/ou le stilbène et/ou la vinylbenzyldiméthylamine et/ou l'éther (4-vinylbenzyl)diméthylaminoéthylique et/ou le N,N-diméthylaminoéthylstyrène et/ou le tert-butoxystyrène et/ou la vinylpyridine et/ou le 1,2-divinylbenzène et/ou le 1,3-divinylbenzène et/ou le 1,4-divinylbenzène.

5. Mélange de caoutchouc réticulable au soufre, **caractérisé en ce qu'**il contient au moins un copolymère, qui est fabriqué par un procédé selon l'une quelconque des revendications 3 ou 4.

6. Utilisation d'un mélange de caoutchouc réticulable au soufre selon la revendication 5 pour la fabrication d'un pneu de véhicule.

7. Utilisation d'un mélange de caoutchouc réticulable au soufre selon la revendication 5 pour la fabrication d'une sangle, d'une courroie ou d'un tuyau.

8. Procédé de fabrication de benzènethiosulfonate de 4-vinylbenzyle par réaction de chlorure de 4-vinylbenzyle avec du benzènethiosulfonate de sodium dans des solvants fortement polaires, notamment de l'eau, à l'aide de catalyseurs de transfert de phase.
